# EUROPEAN PATENT APPLICATION

(11) **EP 2 353 585 A2**
(43) Date of publication of application: **10.08.2011**
(21) Application number: 10174423.3
(22) Date of filing: 27.08.2010
(51) Int. Cl.: A61K 9/00

(54) **Topical drug for treatment and/or prevention of diabetic neuropathy, microangiopathy and diabetic and non diabetic ulcers and wound infection**

(30) Priority: 06.02.2010 US 302089 P; 16.05.2010 US 780905
(71) Applicant: Javid, Dr. Mihan Jafari, Teheran (IR)
(72) Inventor: Javid, Dr. Mihan Jafari, Teheran (IR)
(74) Representative: Wilkinson, Stephen John

(57) **Abstract**

The various embodiments herein provide a topical drug composition for the treatment of diabetes-related complications. According to one embodiment herein, the composition comprises of a compound belonging to a group of chemicals containing borates. Boric acid is used as the base material of the drug. Boric acid is dissolved in saline 0.9% or half saline serum or water, distilled water or boiled water to obtain a boric acid solution with given concentration. The best concentration is 3-5% solution. Alcohol at preset concentration is added to the boric acid solution. According to another embodiment, a method is provided for preparing a topical drug composition for the treatment of diabetes-related disorders.

## Description

### BACKGROUND

### Technical field

The embodiments herein generally relate to the field of treatment of diabetes-related complications such as neuropathy, microangiopathy and ulcers and treatment of resistant wound infections. The embodiments herein more particularly relates to the process of preparation of the drug formulation for the treatment of diabetes-related disorders.

### Description of the Related Art

Borates in chemistry are chemical compounds containing boron oxoanions, with boron in oxidation state +3. The simplest borate ion is the trigonal planar, BO₃³⁻ , although many others are known. BO₃³- forms salts with metallic elements. Boron found in nature is commonly as a borate mineral. Boron is also found combined with silicate to form complex borosilicate minerals such as the tourmalines. Many borates are readily hydrated and contain structural hydroxide groups and should formally be considered as hydroxoborates.

In aqueous solution, borate exists in many forms. In acid and near-neutral conditions, it is boric acid, commonly written as H₃BO₃ but more correctly B(OH)₃. The pKa of boric acid is 9.14 at 25°C. Boric acid does not dissociate in aqueous solution, but is acidic due to its interaction with water molecules, forming tetrahydroxyborate.

Boric acid is the basic material of this drug. Boric acid, also called boracic acid or orthoboric acid or acidum boricum, is a weak acid and often used as an antiseptic, insecticide, flame retardant in nuclear power plants to control the fission rate of uranium and as a precursor of other chemical compounds. It exists in the form of colorless crystals or a white powder and dissolves in water. It has the chemical formula H₃BO₃, Sometimes it is written as B(OH)3. When occurring as a mineral, it is called Sassolite.

The group of chemicals containing borates include basilit B, boracic acid, orthoboric acid, boron trihydroxide, borofax, bortrac, Dia fleamate, flea prufe, trihydroxyborane, trihydroxyborone, three elephant, hydrogen orthoborate, NCl-C56417, sassolite, Acidum boricum.

The water solubility of boric acid is 63.5 g/l at 30°C. Boric acid does not dissociate in aqueous solution, but is acidic due to its interaction with water molecules to form the tetrahydroxyborate ion.

Boric acid can be used as an antiseptic for minor burns or cuts and is sometimes used in dressings or salves or is applied in a very dilute solution as an eye wash in a 1.5% solution (1 tbsp per quart or 15 gr per L) of sterilized water. As an anti-bacterial compound, boric acid can also be used as an acne treatment. Boric acid can be used to treat yeast and fungal infections such as candidiasis (vaginal yeast infections) by inserting a vaginal suppository containing 600 mg of boric acid daily for 14 days or for yeast infection of the male pubic region (jock-itch or strong genital odor) by applying the powder to the skin all over the pubic region for several days to a week. It is also used as prevention of athlete's foot, by inserting powder in the socks or stockings, and in solution can be used to treat some kinds of otitis externa (ear infection) in both humans and animals. The preservative in urine sample bottles (red cap) in the UK is boric acid.

Boric acid was first registered in the US as an insecticide in 1948 for control of cockroaches, termites, fire ants, fleas, silverfish and many other insects. It acts as a stomach poison affecting the insects' metabolism and the dry powder is abrasive to the insects' exoskeleton.

Boric acid is generally considered to be safe for use in household kitchens to control cockroaches and ants. Homemade ant bait can be made by dissolving 1 teaspoon powdered boric acid and 10 teaspoons sugar into 2 cups (∼ 500ml) of water; this mixture can then be absorbed into cotton balls which are left near ant trails. This reportedly will be carried back into the ants' nest, killing any ants that eat it thereby potentially destroying the entire colony.

Boric acid is also made into a paste or gel form as a powerful and effective insecticide much safer to humans than many other insecticides. The paste or gel has attractants in it to attract insects, especially cockroaches that take the bait back to the nest.

In combination with its use as an insecticide, boric acid also prevents and destroys existing wet and dry rot in timbers. It can be used in combination with an ethylene glycol carrier to treat external wood against fungal and insect attack. It is possible to buy borate-impregnated rods for insertion into wood via drill holes where dampness and moisture is known to collect and sit. It is available in a gel form and injectable paste form for treating rot affected wood without the need to replace the timber. Concentrates of borate-based treatments can be used to prevent slime, mycelium and algae growth, even in marine environments.

Colloidal suspensions of nano-particles of boric acid dissolved in petroleum or vegetable oil can form a remarkable lubricant on ceramic or metal surfaces with a coefficient of sliding friction that decreases with increasing pressure to a value ranging from 0.10 to 0.02. Self-lubricating H₃BO₃ films result from a spontaneous chemical reaction between water molecules and B₂O₃ coatings in a humid environment. In bulk-scale, an inverse relationship exists between friction coefficient and Hertzian contact pressure induced by applied load.

The primary industrial use of boric acid is in the manufacture of monofilament fiberglass usually referred to as textile fiberglass. Textile fiberglass is used to reinforce plastics in applications that range from boats to industrial piping to computer circuit boards.

Boric acid is used in nuclear power plants to slow down the rate at which fission is occurring. Fission chain reactions are generally driven by the amount of neutrons present (as products from previous fissions). Natural boron is 20% boron-10 and about 80% boron-11. Boron-10 has a high cross-section for absorption of low energy (thermal) neutrons. By adding more boric acid to the reactor coolant which circulates through the reactor, the probability that a neutron can survive to cause fission is reduced. Therefore, changes in boric acid concentration effectively regulate the rate of fission taking place in the reactor. This method is only used in pressurized water reactors (PWR's). Boron is also dissolved into the spent fuel pools containing used uranium rods. The concentration is high enough to keep neutron multiplication at a minimum.

In the jewelry industry, boric acid is often used in combination with denatured alcohol to reduce surface oxidation and fire scale from forming on metals during annealing and soldering operations.

Boric acid is used the production of the glass in LCD flat panel displays. In electroplating, boric acid is used as part of some proprietary formulas. One such known formula calls for about a 1 to 10 ratio of H₃BO₃ to NiSO₄, a very small portion of sodium lauryl sulfate and a small portion of H₂SO₄. It is also used in the manufacturing of remming mass, a fine silica-containing powder used for producing induction furnace linings and ceramics.
Borates including boric acid have been used since the time of the Greeks for cleaning, preserving food, and other activities. Silly Putty was originally made by adding boric acid to silicone oil.TBE buffer is widely used for the electrophoresis of nucleic acids and has a higher buffer capacity than a TAE buffer. It can be used for DNA and RNA polyacrylamide and agarose gel electrophoresis.

It is used in pyrotechnics to prevent the amide-forming reaction between aluminium and nitrates. A small amount of boric acid is added to the composition to neutralize alkaline amides that can react with the aluminium.

Boric acid dissolved in methanol is popularly used among fire jugglers and fire spinners to create a deep green flame. The white powder is also used to dust down Carrom boards to decrease friction and increase speed of play, Boric acid is added to salt in the curing of cattle hides, calfskins and sheepskins. Used in that way it helps to control bacteria development and also aids in the control of insects. Boric acid is added to borax for use as welding flux by blacksmiths and farriers.

Diabetic ulcer has been dealing with for decades as an unsolved problem. Despite considerable advances in medical and surgical treatment of diabetic patients, diabetic ulcers are the most challenging problem which affects patients' quality of life. Routine diabetic ulcer care costs billions of dollars during year and places tremendous burden on the health care system. More than 80000 amputations performed every year on diabetic ulcers in the USA and 50% of the people with amputation develop ulceration in the contra lateral limb within 18 months. These numbers have not changed much in the past 30 years. In the third millennium every day people become disable and until now there haven't been any effective drugs to prevent such a terrible problem.

Hence there is a need for a topical drug for treatment of Diabetic neuropathy, Diabetic micro angiopathy, Diabetic ulcers and wound infection.

The above mentioned shortcomings, disadvantages and problems are addressed herein and which will be understood by reading and studying the following specification.

### OBJECTIVES OF THE INVENTION

The primary object of the embodiments herein is to provide a topical drug composition for treatment of diabetes related disorders, such as diabetic neuropathy, diabetic angiopathy and micro-angiopathy, diabetic and non-diabetic ulcers and resistant wound infections and other related complications in a short period of time.

Another object of the embodiments herein is to provide a topical drug composition as a solution for bathing the involved limb or involved region.

Another object of the embodiments herein is to provide a topical drug composition as a wet bandage.

Another object of the embodiments herein is to provide a topical drug composition for treatment of use points from other indications such as non diabetic neuropathy and angiopathy, non diabetic ulcers, ischemic ulcers, vasculitis, burger, infected antibiotic resistant ulcers and bedsores.

Another object of the embodiments herein is to provide a topical drug composition for treatment of Arthrosis pain and treatment of neuralgia.

These and other objects and advantages of the present invention will become readily apparent from the following detailed description taken in conjunction with the accompanying drawings.

### SUMMARY

The various embodiments herein provide a topical drug composition for treatment of diabetes related disorders. The drug comprising a compound selected from a group of chemicals, and a solvent selected from a group comprising of normal saline serum, half saline serum, water, boiled water and distilled water. According to one embodiment herein, a composition comprises a compound belonging to a group of chemicals named borates, i.e. boric acid as the base material of the drug. Boric acid is dissolved in saline 0.9% or half saline serum or water or boiled water or distilled water. The best concentration is 3-5% solution. The pharmaceutical composition, as mentioned in the present invention provides a topical treatment for diabetes-related disorders such as diabetic neuropathy, diabetic angiopathy and micro-angiopathy, diabetic ulcers and resistant wound infections and other related diseases in a short period of time.

According to one embodiment herein, a topical drug composition for treatment of diabetes related disorders comprises a compound selected from a group of chemicals containing borates and a solvent selected from a group comprising of serum saline 0.9%, half saline serum , water, boiled water and distilled water. The drug can be prepared in water without the need for boiling or sterilizing. The compound selected from a group of chemicals containing borates is boric acid and the boric acid is dissolved in the selected solvent to achieve a boric acid solution.

According to another embodiment of the present invention, a process of preparing a topical drug for treating diabetes related disorders is provided. The method involves selecting a compound from a group of chemicals containing borates, selecting a solvent from a group comprising normal saline serum, half saline serum, water, boiled water and distilled water and dissolving the selected compound in the selected solvent to prepare a drug solution. The boric acid solution can be prepared in water without any need to boiling or sterilizing for using in patients without open ulcers. The compound selected from a group of chemicals containing borates is boric acid and the drug solution is a boric acid solution. The boric acid solution is prepared as a 0.1-6% solution. Alcohol is added to the boric acid solution at the end. Sometime alcohol should be omitted then boric acid solution is prepared without alcohol. The boric acid solution is prepared at a concentration of 3-5%. Boric acid solution is prepared at a concentration of 3%, 4% and 5%. Boric acid solution is prepared in room air pressure without any requirement of specific pressure.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF THE DRAWINGS

The other objects, features and advantages will occur to those skilled in the art from the following description of the preferred embodiment and the accompanying drawings in which:

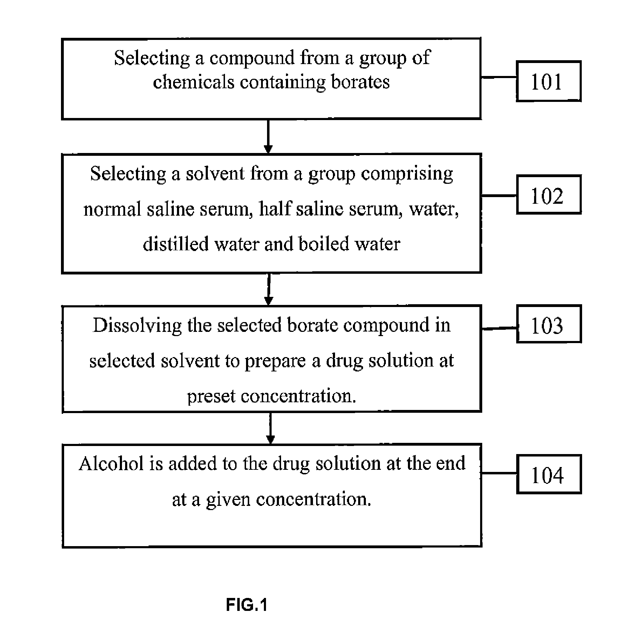

FIG.1 illustrates a flow chart explaining the process of preparing a topical drug composition according to one embodiment of the embodiments herein.

### DETAILED DESCRIPTION OF THE EMBODIMENTS

In the following detailed description, a reference is made to the accompanying drawings that form a part hereof, and in which the specific embodiments that may be practiced is shown by way of illustration. The embodiments are described in sufficient detail to enable those skilled in the art to practice the embodiments and it is to be understood that the logical, mechanical and other changes may be made without departing from the scope of the embodiments. The following detailed description is therefore not to be taken in a limiting sense.

The various embodiments herein provide a topical drug for treatment of diabetes related complications such as Diabetic neuropathy, angiopathy, microangiopathy, diabetic and non-diabetic ulcers and wound infections.

According to one embodiment herein, a topical drug composition for treatment of diabetes related disorders comprises a compound selected from a group of chemicals containing borates and a solvent selected from a group comprising of normal saline, half saline serum , water, distilled water and boiled water. The compound selected from a group of chemicals containing borates is boric acid and the boric acid is dissolved in the selected solvent to achieve a boric acid solution.

The boric acid solution is prepared as a 0.1-6% solution. The boric acid solution is prepared by dissolving boric acid in saline 0.9%. The boric acid solution is prepared by dissolving boric acid in half saline serum. The boric acid solution is prepared by dissolving boric acid in distilled water.

The boric acid solution is prepared in warm to hot saline 0.9 %, half saline serum , water, distilled water and boiled water at a temperature ranging between 30°C-80°C, under atmosphere pressure. The boric acid solution is further added with alcohol at the end. The alcohol is added to the boric acid solution at a concentration of 1-10%. The boric acid solution is prepared at a concentration of 3-5%. The boric acid solution is prepared at concentrations of 3%, 4% and 5%.

According to another embodiment of the present invention, a process of preparing a topical drug for treating diabetes related disorders is provided. The method involves selecting a compound from a group of chemicals containing borates, selecting a solvent from a group comprising normal saline serum, half saline serum, water, distilled water and boiled water and dissolving the selected compound in the selected solvent to prepare a drug solution. The compound selected from a group of chemicals containing borates is boric acid and the drug solution is a boric acid solution. The boric acid solution is prepared as a 0.1-6% solution. Alcohol is added to the boric acid solution at the end.

According to one embodiment, the topical drug for treatment of diabetic complications comprising of a compound related to a group of chemicals, boric acid, alcohol, saline, half saline serum, water, boiled water and distilled water.

The embodiments herein are related to a group of chemicals named borates. This group of chemicals can be used for the treatment of diabetic ulcers resulted from neuropathy and micro angiopathy.

The most specific substance that is focused on in this invention is boric acid as the base material of the drug. The other components required for the preparation of the topical drug are normal saline serum, half saline serum, water, distilled water, boiled water and alcohol.

For the preservation of the prepared drug, it should be kept in an opaque container to keep it away from the light. There is no need of any stabilizer or any other specific situation for the preservation of the drug.

FIG.1 illustrates a flow chart explaining the process of preparing a topical drug composition according to one embodiment of the embodiments herein. With respect to FIG.1, a process of preparing a topical drug for treating diabetes related disorders, involves selecting a compound from a group of chemicals containing borates (101). Then a solvent is selected from a group comprising normal saline serum, half saline serum, water, distilled water and boiled water (102). The selected compound is dissolved in the selected solvent to prepare a drug solution at a preset concentration (103). The compound selected from a group of chemicals containing borates is boric acid and the drug solution is a boric acid solution. The boric acid solution is prepared as a 0.1-6% solution. Alcohol is added to the boric acid solution at the end (104).

The boric acid solution is prepared as a 0.1-6% solution. The boric acid solution is prepared by dissolving boric acid in saline 0.9%. The boric acid solution is prepared by dissolving boric acid in half saline serum. The boric acid solution is prepared by dissolving boric acid in distilled water. The boric acid solution is prepared by dissolving boric acid in boiled water. The boric acid solution can be prepared by dissolving in water without the need for boiling or sterilizing.

The boric acid solution is prepared in warm to hot saline 0.9 %, half saline serum , water, distilled water and boiled water at a temperature ranging between 30°C-80°C, under atmosphere pressure. The boric acid solution is further added with alcohol at the end. The alcohol is added to the boric acid solution at a concentration of 1-10%. Sometime alcohol should be omitted and then boric acid solution is prepared without alcohol. The boric acid solution is prepared at a concentration of 3-5%. The boric acid solution is prepared at a concentration of 3%, 4% and 5%. The boric acid solution is prepared in room air pressure without any requirement of specific pressure.

The water solubility of boric acid is 63.5g/l at 30°C, which is the inherent chemical property of boric acid. Boric acid has proved very low water solubility at room temperature.

The best results of the formulated drug are obtained at the concentration 3-5% solution. There is no difference between concentrations of 3%-5% in the process of treatment. The usage of the formulated topical drug, at a concentration of 3% is safer. Usage of higher concentration depends on the severity of the lesions. The drug so obtained is a very weak acid.

The best way of the treatment is immersing the involved extremity into the solution and using wet bandage or topical drug (lotion) takes more time about one month to treat. The drug can be applied as lotion, cream, patches and gels but it takes more time to treat the pathology.

The drug is used as a solution for bathing the area involved with neuropathy, microangiopathy and ulceration usually one joint upper than the involved area for 5-10 minutes once or twice a day, as a wet bandage prepared with solution and as a topical drug. The drug is also used for other indications such as non diabetic neuropathy and angiopathy, non diabetic ulcers, ischemic ulcers, vasculitis, burger, infected antibiotic resistant ulcers and bedsores.

### EXPERIMENTAL DATA

The formulated topical drug was used in 42 patients with stage 1 and 2 ulcers. In all the patients involved in the experiment, the extremity was cold with impaired sensory response. Two days after starting the treatment, the extremity got normal temperature and sensation and healing of the ulcer completed after one week. The best way of the treatment is immersing the involved extremity into the solution and using wet bandage or topical drug (lotion) takes more time about one month to treat. The drug can be applied as lotion, cream, patches and gels but it takes more time to treat the pathology.

There is no effective drug to treat diabetic microangiopathy and neuropathy to compare with the novel formulated drug comprising boric acid as the base material. All the patients under the study had undergone routine treatments of ulceration such as antibiotic therapy at least for 2 months before using my drug but the ulcers had no response to treatment.

Diabetic ulcers cannot be repaired due to diabetic-neuropathy and microangiopathy. Microangiopathy impairs tissue perfusion and impaired perfusion results in ulceration. Microangiopathy is the result of neuropathy. The new drug has a significant nerve stimulation effect that results in removing debris from the arterioles and reperfusion of the tissue. In fact the ulcer is curable because of reperfusion of the tissue.

Diabetic neuropathy and microangiopathy are curable during 3-7 days and diabetic stage 1 and stage 2 ulcers are curable during 1-2 weeks. Control of the infection in any kind of extended ulcers performs in 3-7 days. Diabetic ulcers are preventable by treatment of diabetic neuropathy and microangiopathy.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the appended claims.

Although the embodiments herein are described with various specific embodiments, it will be obvious for a person skilled in the art to practice the invention with modifications. However, all such modifications are deemed to be within the scope of the claims.

It is also to be understood that the following claims are intended to cover all of the generic and specific features of the embodiments described herein and all the statements of the scope of the embodiments which as a matter of language might be said to fall there between.

## Claims

1. A topical drug composition for treatment and/or prevention of diabetes related disorders, comprising:
a compound selected from a group of chemicals containing borates; and
a solvent selected from a group comprising of saline, half saline serum and distilled water;
wherein the compound selected from a group of chemicals containing borates is boric acid and the boric acid is dissolved in the selected solvent to achieve a boric acid solution with a present concentration.

2. The topical drug composition according to claim 1, wherein the boric acid solution is prepared as a 0.1-6% solution, preferably at a concentration of 3-5%.

3. The topical drug composition according to claim 1, wherein the boric acid solution is prepared by dissolving boric acid in saline 0.9%.

4. The topical drug composition according to claim 1, wherein the boric acid solution is prepared by dissolving boric acid in half saline serum.

5. The topical drug composition according to claim 1, wherein the boric acid solution is prepared by dissolving boric acid in water, preferably boiled water.

6. The topical drug composition according to claim 1, wherein the boric acid solution is prepared in warm hot saline 0.9%, half saline serum, water, boiled water and distilled water at a temperature ranging between 30°C-80°C, under atmosphere pressure.

7. The topical drug composition according to claim 1, wherein the boric acid solution is further added with alcohol at the end, preferably at a concentration of 1-10%.

8. A process of preparing a topical drug for treating diabetes related disorders, comprises:
selecting a compound from a group of chemicals containing borates;
selecting a solvent from a group comprising normal saline serum, half saline serum, water, distilled water and boiled water; and
dissolving the selected compound in the selected solvent to prepare a drug solution;
wherein the compound selected from a group of chemicals containing borates is boric acid and the drug solution is a boric acid solution with a preset concentration.

9. The process of preparing a topical drug according to claim 8, wherein the boric acid solution is prepared as a 0.1-6% solution, preferably at a concentration of 3-5%.

10. The process of preparing a topical drug according to claim 8, wherein the boric acid solution is prepared by dissolving boric acid in saline 0.9%.

11. The process of preparing a topical drug according to claim 8, wherein the boric acid solution is prepared by dissolving boric acid in half saline serum.

12. The process of preparing a topical drug according to claim 8, wherein the boric acid solution is prepared by dissolving boric acid in water, preferably boiled water.

13. The process of preparing a topical drug according to claim 8, wherein the boric acid solution is prepared in warm to hot saline 9.9%, half saline serum, water, distilled water and boiled water at a temperature range of 30°C-80°C, under atmosphere pressure.

14. The process of preparing a topical drug according to claim 8, wherein the alcohol is added to the boric acid solution at a concentration of 1-10%.
